# FASCICULE DE BREVET EUROPEEN

(11) **EP 4 453 071 B1**
(45) Date de publication et mention de la délivrance du brevet: **04.02.2026**
(21) Numéro de dépôt: 22850592.1
(22) Date de dépôt: 20.12.2022
(51) Int. Cl.: C08J 7/16, C09D 4/00, A61L 27/34, A61L 24/06, C08F 292/00

(54) **SUBSTRAT REVETU D'UN POLYMERE OBTENU PAR GREFFAGE D'UNE SOLUTION AQUEUSE DE GREFFAGE**
SUBSTRAT BESCHICHTET MIT EINEM POLYMER ERHALTEN DURCH PFROPFUNG EINER WÄSSRIGEN PFROPFLÖSUNG
SUBSTRATE COATED WITH A POLYMER OBTAINED BY GRAFTING AN AQUEOUS GRAFTING SOLUTION

(30) Priorité: 23.12.2021 FR 2114392
(43) Date de publication de la demande: 30.10.2024
(73) Titulaire: Activ' Biomat, 95957 Roissy Aeroport CDG (FR)
(72) Inventeur: BLANQUAERT, Daniel, 75008 Paris (FR); MIGONNEY, Véronique, 95600 Eaubonne (FR)
(74) Mandataire: CAPRI
(86) Numéro de dépôt international: PCT/FR2022/052443
(87) Numéro de publication internationale: WO 2023/118733

(56) Documents cités:
- WO-A2-2007/141460
- US-A- 5 506 188
- US-A- 6 001 894
- DATABASE CAPLUS [online] 1 April 2008 (2008-04-01), CHEN ZHEN: "Synthesis and application of copolymeric dispersant of acrylic acid-styrene sulfonic sodium-hydroxyethyl methacrylate", XP055942276, Database accession no. 2008:586495

## Description

La présente invention concerne un substrat revêtu d'un polymère obtenu par greffage d'une solution aqueuse de greffage comprenant des monomères dans le but d'obtenir un polymère greffé sur un substrat. L'invention concerne également un procédé de greffage.

L'adhésion ou adhérence cellulaire est une fonction indispensable de l'organisme qui se produit naturellement au cours de la formation de tissus ou d'organes. L'adhésion cellulaire peut se produire entre des cellules et on parle alors d'adhésion inter-cellulaire, soit entre des cellules et un substrat. L'adhésion cellule-substrat se produit dès qu'une cellule se trouve en contact avec une surface naturelle telle qu'une membrane ou encore la matrice extra-cellulaire ou synthétique telle qu'un dispositif médical.

L'adhésion cellulaire peut se produire dans des conditions *in vitro* en laboratoire ou *in vivo* lors de l'implantation d'un dispositif médical. Cette étape est déterminante pour la suite des interactions entre les cellules et la surface d'un dispositif médical.

La prolifération cellulaire est un phénomène naturel au cours duquel les cellules se multiplient pour former des tissus ; elle fait suite à l'adhésion cellulaire.

La nature chimique, physico-chimique ou physique de la surface du substrat modifie très fortement les interactions qui se développent entre les cellules et le substrat au cours de l'adhésion et de la prolifération cellulaires. Dans le but de moduler l'adhésion et la prolifération cellulaire, différents traitements de surface ont été mis au point ; parmi ceux-ci on trouve les traitements pour diminuer voire inhiber l'adhésion et la prolifération des cellules.

Dans l'art antérieur, on connait déjà des techniques de recouvrement ou d'enduction de substrats par des molécules ou macromolécules naturelles ou synthétiques très hydrophiles ou par des hydrogels permettant d'empêcher les interactions entre les cellules et ces substrats et par conséquent diminuer l'adhésion et la prolifération cellulaire. On connait également la modification de substrats par enduction ou fixation d'agents cytotoxiques tels que les ammoniums quaternaires ou les sels d'argent pour empêcher toute adhésion et par conséquent toute prolifération cellulaire. Des méthodes plus simples de type « mécaniques » ont également été proposées pour créer une barrière physique pour empêcher les cellules d'atteindre une surface.

Ces techniques de l'art antérieur ne sont pas satisfaisantes pour les raisons suivantes : le revêtement par des molécules très hydrophiles ou par des hydrogels peut permettre de diminuer l'adhésion des cellules sur un substrat, cependant le revêtement est rarement obtenu par un greffage covalent, et il est fragilisé et son intégrité est difficilement contrôlable lors des étapes de séchage, de stérilisation et plus simplement de conservation et de stockage.

Un autre problème majeur des revêtements greffés réside dans le taux de greffage, qui est souvent insuffisant.

La présente invention a pour but de remédier aux inconvénients précités de l'art antérieur en définissant une solution aqueuse de greffage associant plusieurs monomères aptes à générer un polymère greffé sur un substrat, avec un taux de greffage fortement amélioré.

La solution aqueuse de greffage comprend les monomères suivants :
- du styrène sulfonate de sodium (NaSS),
- de l'acide méthacrylique (MA) ou de l'acide acrylique (AA), et
- de l'hydroxyéthylméthacrylate (HEMA).

Le styrène sulfonate de sodium (NaSS) présente des propriétés permettant d'apporter le caractère glycosaminoglycan-like et une réponse biologique contrôlée de la surface modifiée.

L'acide méthacrylique (MA) ou de l'acide acrylique (AA) présente des propriétés permettant, lorsqu'il est combiné au NaSS de diminuer l'adhésion et la prolifération cellulaires. Il contribue également à apporter le caractère glycosaminoglycan-like à la surface.

Quant à l'hydroxyéthylméthacrylate (HEMA), il présente des propriétés d'hydrophilie qui améliorent la mouillabilité du substrat et permet selon la quantité de groupements « hydroxyle » introduits, de moduler la réponse inflammatoire.

Selon l'invention, on obtient un substrat qui est revêtu d'un polymère obtenu par greffage d'une solution aqueuse de greffage de monomères comprenant un mélange comprenant:
- du styrène sulfonate de sodium (NaSS), et
- de l'acide méthacrylique (MA) ou de l'acide acrylique (AA),

ce mélange comprenant 10 à 90 % en mole de styrène sulfonate de sodium (NaSS) et 10 à 90 % en mole d'acide méthacrylique (MA) ou d'acide acrylique (AA),
dans lequel le substrat est choisi parmi les polyesters, les polymères vinyliques, les polyacryliques et les polyméthacryliques, le PEEK, les silicones, les polymères naturels, les celluloses naturelles ou artificielles, les collagènes, les glycopolymères, les céramiques, les métaux et alliages métalliques, et notamment le Ti et ses alliages et les alliages Ni-Ti, et
la solution aqueuse de greffage comprend, en plus dudit mélange représentant 90 à 99% en mole de la solution aqueuse de greffage, 1 à 10 % en mole d'hydroxyéthylméthacrylate (HEMA).

Il faut bien distinguer les pourcentages de NaSS et de MA ou AA dans le mélange et les pourcentages de mélange et de HEMA dans la solution de greffage.

En plus d'un taux de greffage très élevé avec de I'HEMA entre 1 et 10 % en mole, le polymère greffé est un copolymère capable de minimiser, voire d'inhiber l'adhésion cellulaire de cellules eucaryotes, comme par exemple les cellules ostéoblastes, fibroblastes, kératinocytes, endothéliales, épithéliales sur le substrat et de minimiser ou inhiber la prolifération des cellules adhérentes sur ce même substrat.

Avantageusement, la solution aqueuse de greffage comprend 45 à 49,5 % en mole de styrène sulfonate de sodium (NaSS), 45 à 49,5 % en mole d'acide méthacrylique (MA) ou d'acide acrylique (AA) et 1 à 10 % en mole d'hydroxyéthylméthacrylate (HEMA).

Selon un premier mode de réalisation, la solution aqueuse de greffage peut comprendre 45 % en mole de styrène sulfonate de sodium (NaSS), 45 % en mole d'acide méthacrylique (MA) ou d'acide acrylique (AA) et 10 % en mole d'hydroxyéthylméthacrylate (HEMA).

Selon un second mode de réalisation, la solution aqueuse de greffage peut comprendre 49,5 % en mole de styrène sulfonate de sodium (NaSS), 49,5 % en mole d'acide méthacrylique (MA) ou d'acide acrylique (AA) et 1 % en mole d'hydroxyéthylméthacrylate (HEMA).

Selon un troisième mode de réalisation, la solution aqueuse de greffage peut comprendre 47,5 % en mole de styrène sulfonate de sodium (NaSS), 47,5 % en mole d'acide méthacrylique (MA) ou d'acide acrylique (AA) et 5 % en mole d'hydroxyéthylméthacrylate (HEMA).

Une protection pour de telles solutions aqueuses de greffage pourrait être recherchée.

L'invention définit aussi un procédé de greffage pour appliquer sur un substrat un polymère obtenu par greffage de la solution aqueuse de greffage telle que définie ci-dessus sur un substrat tel que défini ci-dessus, le greffage étant un greffage radicalaire ou un électro-greffage.

L'étape de greffage à proprement parler est de préférence précédée par une étape d'activation de surface. Sur du titane par exemple, le procédé de greffage peut être du type radicalaire avec une étape d'activation de la surface réalisée par oxydation anodique, telle que décrite dans le document WO2017068272. Cette étape préalable d'activation est suivie d'une polymérisation amorcée par voie thermique ou par voie UV. Pour les autres types de substrat, une étape d'activation de la surface est également conseillée, sinon il sera difficile de polymériser à partir de la surface.

L'esprit de l'invention réside dans le fait de faire le choix de la copolymérisation statistique de trois monomères, porteurs de groupements chimiques définis et choisis pour leur spécificité et pour les propriétés qu'ils apporteront à la surface. En d'autres termes, la copolymérisation du NaSS, du MA et de I'HEMA dans des proportions définies permet d'augmenter le taux de greffage, mais également de contrôler l'adhésion et la prolifération cellulaires sur ces surfaces tout en maintenant un contrôle de la réponse inflammatoire.

Ainsi, le greffage d'un copolymère de NaSS, de MA et de HEMA sur du titane ou un alliage de titane permet d'inhiber l'adhésion des cellules ostéoblastes et fibroblastes et de prévenir l'adhérence des cellules et des tissus sur les plaques d'ostéosynthèse destinées à être explantées.

Des mesures du taux de greffage ont été effectuées en laboratoire dans des conditions précises et contrôlées sur différentes pastilles en alliage de Ti. Des pastilles en Ti auraient aussi pu être utilisées.

Tout d'abord, un mélange de NaSS et de MA à 50%/50% en mole (sans HEMA) a été greffé sur une pastille de base P0. Un taux de greffage de base T0 de l'ordre de 5 microgrammes/cm2 a ainsi été mesuré.

Ensuite, trois autres pastilles P1, P2 et P3 ont été revêtues par greffage avec un mélange de NaSS, de MA et de HEMA de l'invention :
- Pastille 1 : greffée avec une solution contenant du NaSS à 49,5 % en mole, du MA à 49,5 % en mole et de HEMA à 1 % en mole, avec un taux de greffage mesuré T1 de l'ordre de 10 microgrammes/cm2,
- Pastille 2 : greffée avec une solution contenant du NaSS à 47,5 % en mole, du MA à 47,5 % en mole et de HEMA à 5 % en mole, avec un taux de greffage mesuré T2 de l'ordre de 15 microgrammes/cm2,
- Pastille 3 : greffée avec une solution contenant du NaSS à 45 % en mole, du MA à 45 % en mole et de HEMA à 10 % en mole, avec un taux de greffage mesuré T3 de l'ordre de 20 microgrammes/cm2.

On a ainsi pu constater que T1 est environ 2 fois supérieur à T0, que T2 est environ 3 fois supérieur à T0 et que T3 est environ 4 fois supérieur à T0. T1 est de la moitié de T3 pour un pourcentage 10 fois inférieur.

On peut donc en conclure qu'un très faible pourcentage de HEMA, à savoir 1%, suffit à augmenter de manière significative (100 %) le taux de greffage d'un mélange de NaSS et de MA. Et avec un pourcentage encore faible de 10 %, le taux de greffage est augmenté, mais de manière moins rapide.

Ce taux de greffage amplifié a bien entendu une influence non négligeable sur l'inhibition de l'adhésion et de la prolifération cellulaires.

Il faut noter que le MA (acide méthacrylique) peut être remplacé par du AA (acide acrylique) dans le mélange de NaSS et de HEMA, avec des résultats équivalents.

## Revendications

1. Substrat revêtu d'un polymère obtenu par greffage d'une solution aqueuse de greffage de monomères comprenant un mélange comprenant:
- du styrène sulfonate de sodium (NaSS), et
- de l'acide méthacrylique (MA) ou de l'acide acrylique (AA),
ce mélange comprenant 10 à 90 % en mole de styrène sulfonate de sodium (NaSS) et 10 à 90 % en mole d'acide méthacrylique (MA) ou d'acide acrylique (AA),
dans lequel le substrat est choisi parmi les polyesters, les polymères vinyliques, les polyacryliques et les polyméthacryliques, le PEEK, les silicones, les polymères naturels, les celluloses naturelles ou artificielles, les collagènes, les glycopolymères, les céramiques, les métaux et alliages métalliques, et notamment le Ti et ses alliages et les alliages Ni-Ti, et
la solution aqueuse de greffage comprend, en plus dudit mélange représentant 90 à 99% en mole de la solution aqueuse de greffage, 1 à 10 % en mole d'hydroxyéthylméthacrylate (HEMA).

2. Substrat selon l'une quelconque des revendications précédentes, dans lequel la solution aqueuse de greffage comprend 45 à 49,5 % en mole de styrène sulfonate de sodium (NaSS), 45 à 49,5 % en mole d'acide méthacrylique (MA) ou d'acide acrylique (AA) et 1 à 10 % en mole d'hydroxyéthylméthacrylate (HEMA).

3. Substrat selon l'une quelconque des revendications précédentes, dans lequel la solution aqueuse de greffage comprend 45 % en mole de styrène sulfonate de sodium (NaSS), 45 % en mole d'acide méthacrylique (MA) ou d'acide acrylique (AA) et 10 % en mole d'hydroxyéthylméthacrylate (HEMA).

4. Substrat selon l'une quelconque des revendications précédentes, dans lequel la solution aqueuse de greffage comprend 49,5 % en mole de styrène sulfonate de sodium (NaSS), 49,5 % en mole d'acide méthacrylique (MA) ou d'acide acrylique (AA) et 1 % en mole d'hydroxyéthylméthacrylate (HEMA).

5. Substrat selon l'une quelconque des revendications précédentes, dans lequel la solution aqueuse de greffage comprend 47,5 % en mole de styrène sulfonate de sodium (NaSS), 47,5 % en mole d'acide méthacrylique (MA) ou d'acide acrylique (AA) et 5 % en mole d'hydroxyéthylméthacrylate (HEMA).

6. Procédé de greffage pour appliquer sur un substrat un polymère obtenu par greffage d'une solution aqueuse de greffage comprenant un mélange de monomères comprenant:
- du styrène sulfonate de sodium (NaSS), et
- de l'acide méthacrylique (MA) ou de l'acide acrylique (AA),
ce mélange de monomères comprenant 10 à 90 % en mole de styrène sulfonate de sodium (NaSS) et 10 à 90 % en mole d'acide méthacrylique (MA) ou d'acide acrylique (AA),
dans lequel la solution aqueuse de greffage comprend, en plus dudit mélange de monomères représentant 90 à 99% en mole de la solution aqueuse de greffage, 1 à 10 % en mole d'hydroxyéthylméthacrylate (HEMA),
dans lequel le greffage est un greffage radicalaire.

7. Procédé de greffage pour appliquer sur un substrat un polymère obtenu par greffage d'une solution aqueuse de greffage comprenant un mélange de monomères comprenant:
- du styrène sulfonate de sodium (NaSS), et
- de l'acide méthacrylique (MA) ou de l'acide acrylique (AA),
ce mélange de monomères comprenant 10 à 90 % en mole de styrène sulfonate de sodium (NaSS) et 10 à 90 % en mole d'acide méthacrylique (MA) ou d'acide acrylique (AA),
dans lequel la solution aqueuse de greffage comprend, en plus dudit mélange de monomères représentant 90 à 99% en mole de la solution aqueuse de greffage, 1 à 10 % en mole d'hydroxyéthylméthacrylate (HEMA),
dans lequel le greffage est un électro-greffage.

8. Procédé de greffage selon la revendication 6 ou 7, dans lequel la solution aqueuse de greffage comprend 45 à 49,5 % en mole de styrène sulfonate de sodium (NaSS), 45 à 49,5 % en mole d'acide méthacrylique (MA) ou d'acide acrylique (AA) et 1 à 10 % en mole d'hydroxyéthylméthacrylate (HEMA).

9. Procédé de greffage selon la revendication 6 ou 7, dans lequel la solution aqueuse de greffage comprend 45 % en mole de styrène sulfonate de sodium (NaSS), 45 % en mole d'acide méthacrylique (MA) ou d'acide acrylique (AA) et 10 % en mole d'hydroxyéthylméthacrylate (HEMA).

10. Procédé de greffage selon la revendication 6 ou 7, dans lequel la solution aqueuse de greffage comprend 49,5 % en mole de styrène sulfonate de sodium (NaSS), 49,5 % en mole d'acide méthacrylique (MA) ou d'acide acrylique (AA) et 1 % en mole d'hydroxyéthylméthacrylate (HEMA).

11. Procédé de greffage selon la revendication 6 ou 7, dans lequel la solution aqueuse de greffage comprend 47,5 % en mole de styrène sulfonate de sodium (NaSS), 47,5 % en mole d'acide méthacrylique (MA) ou d'acide acrylique (AA) et 5 % en mole d'hydroxyéthylméthacrylate (HEMA).

## Patentansprüche

1. Substrat, beschichtet mit einem Polymer, das durch Pfropfen einer wässrigen Pfropflösung von Monomeren erhalten ist, welche ein Gemisch aufweist, das Folgendes aufweist:
- Natriumstyrolsulfonat (NaSS), und
- Methacrylsäure (MA) oder Acrylsäure (AA),
wobei das Gemisch 10 bis 90 Mol-% Natriumstyrolsulfonat (NaSS) und 10 bis 90 Mol-% Methacrylsäure (MA) oder Acrylsäure (AA) umfasst,
wobei das Substrat ausgewählt ist aus Polyestern, Vinylpolymeren, Polyacrylaten und Polymethacrylaten, PEEK, Silikonen, natürlichen Polymeren, natürlichen oder künstlichen Cellulosen, Kollagenen, Glycopolymeren, Keramiken, Metallen und Metalllegierungen, insbesondere Ti und dessen Legierungen sowie Ni-Ti-Legierungen, und
wobei die wässrige Pfropflösung zusätzlich zu dem genannten Gemisch, das 90 bis 99 Mol-% der wässrigen Pfropflösung darstellt, 1 bis 10 Mol-% Hydroxyethylmethacrylat (HEMA) aufweist.

2. Substrat nach einem der vorhergehenden Ansprüche, wobei die wässrige Pfropflösung 45 bis 49,5 Mol-% Natriumstyrolsulfonat (NaSS), 45 bis 49,5 Mol-% Methacrylsäure (MA) oder Acrylsäure (AA) und 1 bis 10 Mol-% Hydroxyethylmethacrylat (HEMA) aufweist.

3. Substrat nach einem der vorhergehenden Ansprüche, wobei die wässrige Pfropflösung 45 Mol-% Natriumstyrolsulfonat (NaSS), 45 Mol-% Methacrylsäure (MA) oder Acrylsäure (AA) und 10 Mol-% Hydroxyethylmethacrylat (HEMA) aufweist.

4. Substrat nach einem der vorhergehenden Ansprüche, wobei die wässrige Pfropflösung 49,5 Mol-% Natriumstyrolsulfonat (NaSS), 49,5 Mol-% Methacrylsäure (MA) oder Acrylsäure (AA) und 1 Mol-% Hydroxyethylmethacrylat (HEMA) aufweist.

5. Substrat nach einem der vorhergehenden Ansprüche, wobei die wässrige Pfropflösung 47,5 Mol-% Natriumstyrolsulfonat (NaSS), 47,5 Mol-% Methacrylsäure (MA) oder Acrylsäure (AA) und 5 Mol-% Hydroxyethylmethacrylat (HEMA) aufweist.

6. Pfropfverfahren zum Aufbringen eines Polymers auf ein Substrat, wobei das Polymer durch Pfropfen einer wässrigen Pfropflösung erhalten wird, die ein Monomergemisch aufweist, das Folgendes umfasst:
- Natriumstyrolsulfonat (NaSS), und
- Methacrylsäure (MA) oder Acrylsäure (AA),
wobei das Monomergemisch 10 bis 90 Mol-% Natriumstyrolsulfonat (NaSS) und 10 bis 90 Mol-% Methacrylsäure (MA) oder Acrylsäure (AA) umfasst,
wobei die wässrige Pfropflösung zusätzlich zu dem genannten Monomergemisch, das 90 bis 99 Mol-% der wässrigen Pfropflösung darstellt, 1 bis 10 Mol-% Hydroxyethylmethacrylat (HEMA) aufweist,
wobei das Pfropfen ein radikalisches Pfropfen ist.

7. Pfropfverfahren zum Aufbringen eines Polymers auf ein Substrat, wobei das Polymer durch Pfropfen einer wässrigen Pfropflösung erhalten wird, die ein Monomergemisch aufweist, das Folgendes umfasst:
- Natriumstyrolsulfonat (NaSS), und
- Methacrylsäure (MA) oder Acrylsäure (AA),
wobei das Monomergemisch 10 bis 90 Mol-% Natriumstyrolsulfonat (NaSS) und 10 bis 90 Mol-% Methacrylsäure (MA) oder Acrylsäure (AA) umfasst,
wobei die wässrige Pfropflösung zusätzlich zu dem genannten Monomergemisch, das 90 bis 99 Mol-% der wässrigen Pfropflösung darstellt, 1 bis 10 Mol-% Hydroxyethylmethacrylat (HEMA) aufweist,
wobei das Pfropfen ein elektrochemisches Pfropfen ist.

8. Pfropfverfahren nach Anspruch 6 oder 7, wobei die wässrige Pfropflösung 45 bis 49,5 Mol-% Natriumstyrolsulfonat (NaSS), 45 bis 49,5 Mol-% Methacrylsäure (MA) oder Acrylsäure (AA) und 1 bis 10 Mol-% Hydroxyethylmethacrylat (HEMA) aufweist.

9. Pfropfverfahren nach Anspruch 6 oder 7, wobei die wässrige Pfropflösung 45 Mol-% Natriumstyrolsulfonat (NaSS), 45 Mol-% Methacrylsäure (MA) oder Acrylsäure (AA) und 10 Mol-% Hydroxyethylmethacrylat (HEMA) aufweist.

10. Pfropfverfahren nach Anspruch 6 oder 7, wobei die wässrige Pfropflösung 49,5 Mol-% Natriumstyrolsulfonat (NaSS), 49,5 Mol-% Methacrylsäure (MA) oder Acrylsäure (AA) und 1 Mol-% Hydroxyethylmethacrylat (HEMA) aufweist.

11. Pfropfverfahren nach Anspruch 6 oder 7, wobei die wässrige Pfropflösung 47,5 Mol-% Natriumstyrolsulfonat (NaSS), 47,5 Mol-% Methacrylsäure (MA) oder Acrylsäure (AA) und 5 Mol-% Hydroxyethylmethacrylat (HEMA) aufweist.

## Claims

1. Substrate coated with a polymer obtained by grafting an aqueous monomer grafting solution comprising a mixture that comprises:
- sodium styrene sulphonate (NaSS), and
- methacrylic acid (MA) or acrylic acid (AA), and
this mixture comprising 10 to 90mol% of sodium styrene sulphonate (NaSS) and 10 to 90mol% of methacrylic acid (MA) or acrylic acid (AA), wherein the substrate is selected from among polyesters, vinyl polymers, polyacrylics and polymethacrylics, PEEK, silicones, natural polymers, natural or artificial celluloses, collagens, glycopolymers, ceramics, metals and metal alloys, and in particular Ti and alloys thereof and Ni-Ti alloys, and
the aqueous grafting solution comprises, in addition to said mixture which represents 90 to 99mol% of the aqueous grafting solution, 1 to 10mol% of hydroxyethylmethacrylate (HEMA).

2. Substrate according to any one of the preceding claims, wherein the aqueous grafting solution comprises 45 to 49.5mol% of sodium styrene sulphonate (NaSS), 45 to 49.5mol% of methacrylic acid (MA) or acrylic acid (AA), and 1 to 10mol% of hydroxyethylmethacrylate (HEMA).

3. Substrate according to any one of the preceding claims, wherein the aqueous grafting solution can comprise 45%mol% of sodium styrene sulphonate (NaSS), 45mol% of methacrylic acid (MA) or acrylic acid (AA) and 10mol% of hydroxyethylmethacrylate (HEMA).

4. Substrate according to any one of the preceding claims, wherein the aqueous grafting solution comprises 49.5mol% of sodium styrene sulphonate (NaSS), 49.5mol% of methacrylic acid (MA) or acrylic acid (AA), and 1mol% of hydroxyethylmethacrylate (HEMA).

5. Substrate according to any one of the preceding claims, wherein the aqueous grafting solution comprises 47.5%mol% of sodium styrene sulphonate (NaSS), 47.5mol% of methacrylic acid (MA) or acrylic acid (AA) and 5mol% of hydroxyethylmethacrylate (HEMA).

6. Grafting method to apply, on a substrate, a polymer obtained by grafting an aqueous grafting solution, comprising a monomer mixture that comprises:
- sodium styrene sulphonate (NaSS), and
- methacrylic acid (MA) or acrylic acid (AA),
this monomer mixture comprising 10 to 90mol% of sodium styrene sulphonate (NaSS) and 10 to 90mol% of methacrylic acid (MA) or acrylic acid (AA),
wherein the aqueous grafting solution comprises, in addition to said monomer mixture which represents 90 to 99mol% of the aqueous grafting solution, 1 to 10mol% of hydroxyethylmethacrylate (HEMA), wherein the grafting is a radical grafting.

7. Grafting method to apply, on a substrate, a polymer obtained by grafting an aqueous grafting solution, comprising a monomer mixture that comprises:
- sodium styrene sulphonate (NaSS), and
- methacrylic acid (MA) or acrylic acid (AA),
this monomer mixture comprising 10 to 90mol% of sodium styrene sulphonate (NaSS) and 10 to 90mol% of methacrylic acid (MA) or acrylic acid (AA),
wherein the aqueous grafting solution comprises, in addition to said monomer mixture which represents 90 to 99mol% of the aqueous grafting solution, 1 to 10mol% of hydroxyethylmethacrylate (HEMA), wherein the grafting is an electrografting.

8. Grafting method according to claim 6 or 7, wherein the aqueous grafting solution comprises 45 to 49.5mol% of sodium styrene sulphonate (NaSS), 45 to 49.5mol% of methacrylic acid (MA) or acrylic acid (AA), and 1 to 10mol% of hydroxyethylmethacrylate (HEMA).

9. Grafting method according to claim 6 or 7, wherein the aqueous grafting solution comprises 45%mol% of sodium styrene sulphonate (NaSS), 45mol% of methacrylic acid (MA) or acrylic acid (AA) and 10mol% of hydroxyethylmethacrylate (HEMA).

10. Grafting method according to claim 6 or 7, wherein the aqueous grafting solution comprises 49.5mol% of sodium styrene sulphonate (NaSS), 49.5mol% of methacrylic acid (MA) or acrylic acid (AA), and 1mol% of hydroxyethylmethacrylate (HEMA).

11. Grafting method according to claim 6 or 7, wherein the aqueous grafting solution comprises 47.5mol% of sodium styrene sulphonate (NaSS), 47.5mol% of methacrylic acid (MA) or acrylic acid (AA) and 5mol% of hydroxyethylmethacrylate (HEMA).
